(19) Europäisches Patentamt
European Patent Office
Office européen des brevets

(11) Veröffentlichungsnummer: **0 267 321**
**B1**

(12) # EUROPÄISCHE PATENTSCHRIFT

(45) Veröffentlichungstag der Patentschrift: **28.02.90**

(51) Int. Cl.⁴: **A61K 31/19**

(21) Anmeldenummer: **86115843.4**

(22) Anmeldetag: **14.11.86**

(54) **Ibuprofen enthaltendes Arzneimittel.**

(43) Veröffentlichungstag der Anmeldung:
**18.05.88 Patentblatt 88/20**

(45) Bekanntmachung des Hinweises auf die Patenterteilung:
**28.02.90 Patentblatt 90/9**

(84) Benannte Vertragsstaaten:
**AT BE CH DE ES FR GB GR IT LI LU NL SE**

(56) Entgegenhaltungen:
**CHEMICAL ABSTRACTS, Band 106, Nr. 9, 2. März 1987, Seite 574, Nr. 66922r, Columbus, Ohio, US; & JP-A-61 210 049 (DAICEL CHEMICAL INDUSTRIES, LTD.) 18.09.1986**
**MARTINDALE - THE EXTRA PHARMACOPOEIA, 28. Auflage, Ausgegeben von J.E.F.Reynolds et al., 1982, Seite 1979, The Pharmaceutical Press, London, GB;**
**J. PHARM. PHARMAC., Band 28, Nr. 3, 1976, Seiten 256-257; S.S.ADAMS et al.: "Pharmacological differences between the optical isomers of ibuprofen: evidence for metabolic inversion of the (-)-isomer"**
**JOURNAL OF PHARMACEUTICAL SCIENCES, Band 73, Nr. 11, November 1984, Seiten 1542-1544, American Pharmaceutical Association; E.J.D.LEE et al.: "Liquid chromatographic determination and plasma concentration profile of optical isomers of ibuprofen in humans" LENCHIK et al.: "Effect of nature of halide ions on oligomerization of hexafluoropropylene oxid 000**
**BIOCHEMICAL PHARMACOLOGY, Band 35, Nr. 19, 1.**

(73) Patentinhaber: **MEDICE Chem.-Pharm. Fabrik Pütter GmbH & Co. KG, Kuhloweg 37-39, D-5860 Iserlohn/Westfalen(DE)**

(72) Erfinder: **Loew, Dieter, PD Dr. Dr., Katernberger Strasse 255, D-5600 Wuppertal(DE)**
Erfinder: **Schuster, Otto, Dr., PAZ In der Schildwacht, D-6000 Frankfurt(DE)**
Erfinder: **Lukas, J., Dr., PAZ In der Schildwacht, D-6000 Frankfurt(DE)**

(74) Vertreter: **Hansen, Bernd, Dr.rer.nat. et al, Hoffmann, Eitle & Partner Patentanwälte Arabellastrasse 4 Postfach 81 04 20, D-8000 München 81(DE)**

(56) Entgegenhaltungen: (Fortsetzung)
**Oktober 1986, Seiten 3403-3405, Pergamon Journals Ltd.; K.WILLIAMS et al.: "The stereoselective uptake of ibuprofen enantiomers into adipose tissue"**
**Selecta Suppl. 35/1985, S. 22**

**EP 0 267 321 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Nicht steroidale Antirheumatika (NSAR) werden in großem Umfang in der Rheumatherapie eingesetzt. Es liegen umfangreiche Ergebnisse darüber vor, daß die NSAR Hemmstoffe bei der Bildung von Prostaglandinen sind und die therapeutische Wirksamkeit im Zusammenhang mit der Hemmwirkung der Prostaglandinbildung korreliert.

Alle NSAR weisen neben den gewünschten Wirkungen, nämlich den antiphlogistischen, analgetischen und zum Teil antipyrretischen Eigenschaften unerwünschte Nebenwirkungen auf. Dazu gehören eine Beeinträchtigung des Magen- Darmtraktes, die Ausbildung von Magenulcera, Diarrhoen, eine Natriumretention, Ausbildung von Ödemen, Nierenschäden sowie Einwirkungen auf das zentrale Nervensystem und den Respirationstrakt. Außer diesen allgemeinen Nebenwirkungen können durch NSAR auch spezifische unerwünschte Nebenwirkungen, insbesondere Allergien, verursacht werden.

Die NSAR, die insbesondere bei rheumatischen Erkrankungen verschrieben werden, müssen, um wirksam zu werden und dem Patienten die gewünschte Erleichterung zu verschaffen, in einer ausreichenden Menge dosiert werden. Bei hohen Dosierungen, wie sie häufig für die akute Schmerzbekämpfung erforderlich ist, und bei einer Dauermedikation, wie sie insbesondere bei Rheumatikern oft unvermeidlich ist, erhöhen sich dadurch die Gefahren durch die unerwünschten Nebenwirkungen.

Ibuprofen, das ist 2-(4-Isobutylphenyl)-propionsäure mit der Strukturformel

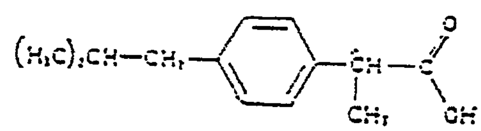

ist ein seit Jahren bewährtes NSAR aus der Gruppe der Phenylpropionsäurederivate, das sich über die Prostaglandinsynthesehemmung in den tierexperimentellen Entzündungsmodellen als wirksam erwiesen hat. In der Humantherapie reduziert Ibuprofen entzündlich bedingte Schmerzen, Schwellungen und Fieber. Es zeigt die für NSAR üblichen unerwünschten Nebenwirkungen. Der empfohlene Dosisbereich für die orale und rektale Darreichungsform liegt bei maximalen Einzeldosen von 800 mg zwischen 1200 und maximal 2400 mg Ibuprofen per die und ist damit, auch im Vergleich zu anderen NSAR, insbesondere etwa zu den bewährten Verbindungen Diclofenac und Indometacin, für die mittlere Tagesdosen von 0,2 bzw. 0,1 g empfohlen werden, während für Ibuprofen die empfohlene mittlere Tagesdosis bei mehr als 1 g liegt, verhältnismäßig hoch. Entsprechend groß sind daher auch die Einzeldosierungsformen als Tablette oder Dragee, die 200 mg nicht unterschreiten und in der Regel bei 400 bis 600 mg liegen, weil eine ausreichende Menge des Wirkstoffes erforderlich ist, um den maximalen Plasmaspiegel zu erreichen. Diese großen Tabletten oder Dragees werden als Nachteil angesehen, weil sie oft schwer zu schlucken sind. Kleinere Dosierungsformen, von denen dann mehrere oder die häufiger eingenommen werden müssen, sind wegen der dadurch bedingten Belastung des Patienten nicht sinnvoll. Die großen Dosierungsformen erschweren auch eine Retardformulierung, weil durch das Retardierungsmittel zusätzliche Stoffe in die Dosierungsform eingearbeitet werden müssen.

Ibuprofen hat ein asymmetrisches Kohlenstoffatom und liegt in der therapeutisch angewendeten Form als Racemat vor. Wie für zahlreiche Arzneimittel-Wirkstoffe mit einem oder mehreren asymmetrischen C-Atom(en) bekannt ist, ist die eine enantiomere Form häufig wirksamer oder sogar sehr viel wirksamer als die andere enantiomere Form, und in Einzelfällen ist die pharmakologische Aktivität überhaupt nur einem der Enantiomeren zuzuschreiben. Es ist bekannt, daß (R)-(–)-Ibuprofen wesentlich weniger pharmakologisch aktiv ist als (S)-(+)-Ibuprofen. Da jedoch das unwirksame (R)-(–)-Enantiomer im menschlichen Organismus in das wirksame (S)-(+)-Enantiomer umgewandelt wird, was durch Messungen der mit dem Urin ausgeschiedenen Ibuprofenmetabolide belegt wurde, hat man bisher keinen therapeutischen Vorteil bezüglich der Anwendung der (S)-(+)-Form anstelle des Racemats abgeleitet und eine Auftrennung der rechtsdrehenden und der linksdrehenden Form nicht für sinnvoll gehalten, vergl. hiezu das in Selecta-Suppl. 35 (1985), S. 22 publizierte Vorurteil sowie die pharmakologischen Studien zur Biopharmazie des Ibuprofen und seiner Enantiomere bzw. des Razemats, welche beispielsweise in C. A. 106 (1987), Nr. 66922r, J. Pharm. Sci, 73, (1984), S. 1542–44, Biochem. Pharmacol. 35 (1986), S. 3403–3405 oder in J. Pharmacol., 28 (1976), S. 256–7 beschrieben sind.

Es wurde nun gefunden, daß sich entgegen der etablierten Auffassung, wonach das Ibuprofen-Racemat die geeignetste therapeutisch wirksame Form darstellt, weil das unwirksame (R)-(–)-Enantiomer im menschlichen Organismus in das wirksame (S)-(+)-Enantiomer umgewandelt wird, die (S)-(+)-Form des Ibuprofens, also in Abwesenheit der (R)-(–)-Form, ein wesentlich größeres pharmakologisches Potential besitzt, als zu vermuten war. Auf diese Erkenntnis baut die vorliegende Erfindung auf.

Die Erfindung betrifft ein die in den Ansprüchen gekennzeichneten, das (S)-(+)-Ibuprofen enthaltende Arzneimittel und die Verwendung des Ibuprofens in Form des (S)-(+)-Enantiomers zur Herstellung solcher Arzneimittel. Es war nicht zu erwarten, daß durch die alleinige Verwendung des (S)-(+)-Enantiomers eine wesentliche Dosisverminderung möglich ist, da ja bekannt war, daß an sich weitgehend unwirksame (R)-(–)-Ibuprofen im menschlichen Organismus in das wirksame (S)-(+)-Ibuprofen umgelagert wird.

Überraschenderweise wurde aber gefunden, daß bei alleiniger Verwendung von (S)-(+)-Ibuprofen als Ibuprofen-Wirkstoff in den beanspruchten Mitteln die Dosis nicht nur halbiert werden kann, sondern daß man sogar mit weniger als der halben Dosis der (S)-(+)-Form im Vergleich zum Racemat eine gleiche analgetische Wirkung erzielt. Dieses Ergebnis ist aufgrund der bisherigen Erkenntnisse über den Wirkungsmechanismus der NSAR im einzelnen und bei Ibuprofen im besonderen außerordentlich überraschend. Diese Erkenntnisse stammen aus Anlagesie-Tests beim Affen, der aufgrund seiner phylogenetischen Stellung dem Menschen im Stoffwechselverhalten am nächsten kommt. Sie konnten auch am Menschen selbst bestätigt werden.

Dieses überraschende Ergebnis könnte sich dadurch erklären, daß die bei der Inversion der inaktiven (R)-(–)-Form im menschlichen Organismus entstehende (S)-(+)-Form aufgrund seiner Bildung in der Leber und der gänzlich anderen Anflutungskinetik schnell metabolisch inaktiviert und ausgeschieden wird. Deshalb kann die metabolisch aus der (R)-(–)-Form entstandene (S)-(+)-Form nicht mehr durch die erforderlichen Verteilungsvorgänge an den Wirkort, nämlich das entzündete Gewebe, gelangen. Andererseits kann das Vorliegen der (R)-(–)-Form die Verteilung der (S)-(+)-Form in das entzündete Gewebe zurückdrängen.

Um eine ausreichende pharmakologische Wirkung beim Menschen zu erzielen, ist es entscheidend, daß das reine (S)-(+)-Enantiomer eingesetzt wird, weil nur dann im Blutplasma schnell genug eine ausreichend hohe Konzentration erreicht wird, die für eine schnelle Verteilung bis an den Wirkort erforderlich ist. Nach der Gabe der (S)-(+)-Form wird eine wesentlich höhere Konzentration am Wirkort erreicht als nach der Applikation des Racemats bzw. der (R)-(-)-Form.

Dies ist von eminenter therapeutischer Bedeutung; denn die erfindungsgemäße Verringerung der wirksamen Dosis verringert natürlich auch die unerwünschten Nebenwirkungen. Das quasi als Ballast im Racemat enthaltene (R)-(-)-Racemat fällt fort und damit auch die Nebenwirkungen, die durch diesen 50%-igen Anteil im Racemat verursacht wurden. Darüberhinaus wird auch das bei der im Organismus partiell stattfindenden Inversion der (R)-(-)-Form in die (S)-(+)-Form beanspruchte spezifische Enzymsystem (Co-A-Derivat), welches bei der Metabolisierung der (S)-(+)-Form nicht benötigt wird, nicht mehr beansprucht. Da dieses Enzymsystem für lebensnotwendige Stoffwechsel- und Entgiftungsvorgänge wesentlich ist, ist es vorteilhaft, daß es bei der Verwendung des (S)-(+)-Racemates als alleiniger Ibuprofen-Wirkstoff nicht beansprucht wird und somit voll für die Entgiftungs- und Stoffwechselvorgänge zur Verfügung bleibt.

Es liegt hier ein "umgekehrter" Synergismus vor. (S)-(+)-Ibuprofen weist in der halben Dosis eine größere Akivität auf als das entsprechende Racemat. Dadurch resultiert die Möglichkeit, in einer Dosierungsform mit weniger als der halben Menge auszukommen, die bisher zur Erzielung eines gleichen Ergebnisses beim Einsatz des Racemats erzielt wurde. Die Einzeldosierungsform, also Tabletten oder Dragees etc., können entsprechend kleiner hergestellt werden. Umgekehrt kann bei gleicher Tabletten- bzw. Drageegröße die doppelte Menge des aktiven Wirkstoffes formuliert werden. Da aber durch das bisherige "Mitschleppen" der unwirksamen (R)-(-)-Form im Racemat nicht nur das bisherige Volumen der Tabletten oder Dragees groß gehalten werden mußte, sondern das mitgeschleppte (R)-(-)-Enantiomer auch in Konkurrenz zum gleichzeitig vorliegenden wirksamen (S)-(+)-Enantiomer tritt, kann die tatsächliche Menge des Wirkstoffes noch unterhalb der halben Menge des Racemates liegen.

Anstelle der bisher für erforderlich gehaltenen durchschnittlichen Tagesdosen von 1200 bis 2400 mg können diese Dosen mindestens halbiert werden. Die mögliche Verkleinerung der Dosierungsform ermöglicht aber auch die bisher erschwerte und in der Praxis nicht durchgeführte Retardirung. Auch Zwei-Stufen-Tabletten, bei denen das (S)-(+)-Enantiomer in Retardform in Kombination mit einer nicht-retardierten Form vorliegt, lassen sich nunmehr einfacher formulieren, ohne daß solche Dosierungsformen zu voluminös und darum für den Patienten nicht mehr applikabel werden.

Die Formulierung von (S)-(+)-Ibuprofen gemäß Erfindung zu Tabletten, Dragees und anderen für die orale Applikation geeigneten Retardformulierungen erfolgt in üblicher Weise, wobei die bekannten Trägerstoffe und Verdünnungsmittel verwendet werden. Solche Formulierungen sind aus der Galenik bekannt und können auch bei der vorliegenden Erfindung angewendet werden. Dies gilt in gleicher Weise auch für die übrigen beanspruchten Formulierungen für die rektale oder parenterale Verabreichung bzw. für Salben oder Gele zur kutanen Applikation. Allen galenischen Zubereitungen ist aber gemeinsam, daß sie, da sie Ibuprofen nur als S)-(+)-Enantiomer enthalten, mit einer geringeren Menge des aktiven Wirkstoffes auskommen, oder daß bei gleicher Wirkstoffkonzentration im Vergleich zu der entsprechenden Racematform eine mehr als die doppelte pharmakologische Wirksamkeit erreicht wird.

Für injizierbare Zubereitungen wird das (S)-(+)-Ibuprofen in Kombination mit einer Aminosäure zu formulieren. Hierdurch wird die Solubilisierung verbessert.

Die Gewinnung von (S)-(+)-Ibuprofen erfolgt durch eine übliche Racematspaltung. Hierzu kann man beispielsweise optisch aktives Phenylethylamin verwenden. (R)-(+)-Phenylethylammonium-(R)-(-)-ibuprofemat und (S)-(-)-Phenylethylammonium-(S)-(+)-ibuprofemat zeigen eine geringere Löslichkeit als ihre Diastereomeren und können deshalb von diesen abgetrennt werden. Durch mehrmaliges Umkristallisieren der schwerer löslichen Diastereomeren aus Ethylacetat/Ethanolgemischen können die Diastereomeren gereinigt werden. Durch Säurespaltung mit beispielsweise Salzsäure und anschließender Extraktion mit Ether wird das jeweilige Ibuprofenenantiomer aus der diastereomeren Verbindung befreit und in reiner Form dargestellt.

Die nachfolgenden Beispiele beschreiben einige erfindungsgemäße Rezepturen:

| Beispiel 1 | |
|---|---|
| Ibuprofen, Tabletten | |
| 1 Tablette enthält in mg | |
| (S)-(+)-Ibuprofen* | 300,0 |
| Unlösliches Polyvinylpyrrolidon | 20,0 |
| Mikrokristalline Cellulose | 150,0 |
| Magnesiumstearat | 10,0 |
| Die Tablette kann auch als Filmtablette hergestellt werden. | |
| * Der Wirkstoff kann variiert werden von 20 bis 800 mg/Tablette. Die Hilfsstoffe werden entsprechend reduziert bzw. erhöht. | |

| Beispiel 2 | |
|---|---|
| Ibuprofen, Kapseln | |
| 1 Retardkapsel enthält in mg | |
| (S)-(+)-Ibuprofen* | 200,0 |
| (S)-(+)-Ibuprofen retard* | 200,0 |

Die Retardpellets des (S)-(+)-Ibuprofen enthalten außer den 200 mg Wirkstoff

Saccharose
Maisstärke
Stearinsäure
Poly( carboxymethyl)stärke, Natriumsalz
Poly(1-vinyl-2-pyrrolidon)
Schellack
Talkum

* Der Wirkstoff in der nichtretardierten sowie in der retardierten Form muß nicht nur im Verhältnis 1:1 vorliegen, er kann ebenso im Verhältnis 1:0,5 – 1:4 eingesetzt werden. Entsprechend diesen Änderungen des Wirkstoffs ändern sich die benötigten Hilfsstoffe in diesen Pellets.

| Beispiel 3 | |
|---|---|
| Ibuprofen, Suppositorien | |
| 1 Suppositorium enthält in mg | |
| (S)-(+)-Ibuprofen* | 450,0 |
| Hartfett | 1891,0 |
| Tocopherol | 9,0 |
| * Der Wirkstoff kann in dieser Arzneiform von 20 bis 800 mg variiert werden, entsprechend ändert sich die notwendige Menge Hartfett, um zu einem Suppositorium von 2,3 g zu gelangen. | |

| Beispiel 4 | |
|---|---|
| Ibuprofen, Injektionslösung | |
| In einer 3 ml fassenden Ampulle sind 2 ml Lösung folgender Zusammensetzung enthalten: | |
| (S)-(+)-Ibuprofen* | 300 mg |
| dl-Lysin | 212 mg |

| Beispiel 5 | |
|---|---|
| Ibuprofen, Injektionslösung | |
| In einer 3 ml fassenden Ampulle sind 2 ml Lösung folgender Zusammensetzung enthalten: | |
| (S)-(+)-Ibuprofen* | 300,0 mg |
| Meglumin | 258,7 mg |
| *Der Wirkstoff bei diesen beiden Arzneiformen kann variiert werden von 200 bis 500 mg, entsprechend müssen die Hilfsstoffe Lysin und Meglumin reduziert bzw. erhöht werden. Gegebenenfalls muss auch das Lösungsmittel Wasser erhöht oder die Größe der Ampulle variiert werden. Der pH-Wert der Injektionslösung sollte zwischen 7,3 und 7,8 liegen. | |

Beispiel 6

Für die kutane Applikation wird eine Salbe oder ein Gel in einer Konzentration von 3 bis 8 Gew.% (S)-(+)-Ibuprofen hergestellt.

Bewertung der analgetischen Aktivität von Ibuprofen

Bei diesem Versuch wurden die afferenten Nerven der Füße von weiblichen Rhesusaffen elektrisch stimuliert. Für die Untersuchung wurden vier erwachsene weibliche Rhesusaffen (macaca mulatta) herangezogen.

Es wurden die folgenden Wirkstoffe verwendet:

(S)-(+)-Ibuprofen
(R)-(-)-Ibuprofen
(±)-Ibu rofen
Acetylsalicylsäure

Methode:

Die Affen waren dahingehend erzogen worden, daß sie während der Versuche in Sesseln saßen und einen Schuh aus Aluminiumfolie trugen.

An den Versuchstagen wurden die Affen in die Stühle gesetzt. Ein Fuß eines jeden Affen wurde mit Elektrodengel eingeschmiert und der Aluminiumschuh darübergezogen.

Ein Grass-Stimulator (S 88) wurde über einen Mehrwegschalter mit den Affen verbunden (die positive Leitung wurde an den Aluminiumschuh angeschlossen, die negative Leitung an den Metallrahmen des Stuhls). Es erfolgte eine elektrische Stimulierung von jeweils 2 Sekunden mit einer Frequenz von 60 Hz (die Stimulusmagnitude wurde verändert durch Einstellung der dem Stimulator zugeführten Spannung und der ensprechende Strom wurde mittels eines in Serie geschalteten Amperemeters gemessen).

Es wurde der Stimulus festgestellt, der erforderlich war, eine Reizschwelle zu erreichen. Hierzu wurde die Spannung stufenweise erhöht. Die Schwellspannung wurde als die niedrigste Spannung angesehen, bei welcher eine Krümmung der Zehen oder ein Verdrehen des Fußes erfolgte.

Vor der Applikation der Arzneimittel ließ man die Tiere wenigstens eine Stunde aklimatisieren. Während dieses Zeitraums wurde die Schwellspannung in 10-minütigen Abständen bestimmt, bis übereinstimmende Ablesungen erhalten wurden.

Im Anschluß an die Aklimatisierungsperiode wurden der Trägerstoff oder der Wirkstoff oral verabreicht. Die Schwellspannung, die erforderlich war, eine Reizreaktion am Fuß bei jedem Affen zu bewirken, wurde in 30-minütigen Intervallen während 3 Stunden und dann nach 4 und 5 Stunden nach der Verabreichung des Wirkstoffes bestimmt.

Die Beobachter und die Stimulierungsvorrichtung waren hinter einem Einwegspiegel verborgen.

Jedes Tier wurde mit den folgenden Wirkstoffen in den angegebenen Mengen behandelt:

| | | | |
|---|---|---|---|
| 1. | (S)-(+)-Ibuprofen | 50 | mg/kg |
| 2. | (R)-(−)-Ibuprofen | 50 | mg/kg |
| 3. | (±)-Ibuprofen | 50 | mg/kg |
| 4. | Acetylsalicylsäure | 100 | mg/kg |
| 5. | Träger (0,5% Carboxymethylcellulose) | | |

Zwischen den einzelnen Tests wurde ein Intervall von wenigstens einer Woche eingelegt.

Die Wirkstoffe 1 bis 4 wurden zu oralen Verabreichungen zubereitet, indem man sie in 1,0% w/v Tragacanth suspendierte. Die Arzneimittel wurden unmittelbar vor der Verwendung hergestellt und in einer Dosis von 4 ml/kg verabreicht.

Die erzielten Ergebnisse werden in Tabelle 1 und in der Fig. 1 gezeigt.

Träger

Orale Verabreichung des Trägers (1,0% Tragacanth) ergab eine geringe Verringerung der Schwellspannung, um eine Ansprechung zu erzielen.

Acetylsalicylsäure

Nach der Verabreichung von 100 mg/kg Acetylsalicylsäure wurde die Schwellspannung, die für eine Reizreaktion erforderlich war, um annäherend 80% erhöht (Fig. 1). Dieses Niveau der Analgesie wurde innerhalb von 2 Stunden nach der Verabreichung des Wirkstoffes erzielt und blieb während der Versuchsdauer oberhalb 60% erhalten. Eine Analge sie wurde angenommen, wenn die Schwellspannung 20% oberhalb der Schwelle, die nach der Verabreichung des Trägers allein erzielt wurde, lag.

Ibuprofen

Orale Verabreichung von (R)-(-)-Ibuprofen (50 mg/kg) ergaben keine wesentliche Veränderung der Schwellspannung. Dagegen ergab eine orale Verabreichung von (S)-(+)-Ibuprofen (50 mg/kg) eine Erhöhung der Schwellspannung von annähernd 50% (Fig. 1). Dies wurde 90 Minuten nach der Verabreichung des Wirkstoffes erzielt und die Schwellspannung blieb während der restlichen Versuchsdauer erhöht. Nach der Verabreichung von Ibuprofenracemat (50 mg/kg) wurde die Schwellspannung um etwa 15% erhöht. Dies wurde innerhalb von 90 Minuten erreicht und dieses Niveau blieb während der Versuchsdauer erhalten. Obwohl das Racemat die Schwellspannung nur um 15% erhöhte, wurde hier eine analgetische Wirkung angenommen, weil die Schwellspannung, nachdem nur der Träger verabreicht worden war, abnahm. Der Unterschied zwischen der mittleren Veränderung zwischen Ibuprofenracemat und Träger betrug deshalb 90 Minuten nach der Verabreichung des Arzneimittels für jeden Zeitpunkt mehr als 20% mit der Ausnahme nach 150 Minuten (siehe Fig. 1).

Beurteilung der Versuche

Bei den Versuchstieren ergab (S)-(+)-Ibuprofen eine merkliche analgetische Wirkung, die mehrere Stunden beibehalten wurde. Das (R)-(-)-Isomer war inaktiv und das Racemat hatte eine schwache, aber lang anhaltende analgetische Wirkung.

Tabelle 1

Prozentuale Veränderung der Spannung, die erforderlich ist, um eine Reizreaktion bei den weiblichen Rhesusaffen zu erzielen

| Behandlung | Orale Dosis (mg/kg) | Mittlere prozentuale Veränderung der Schwellspannung zu unterschiedlichen Zeiten (h) nach der Verabreichung des Wirkstoffs | | | | | | | |
|---|---|---|---|---|---|---|---|---|---|
| | | 0,5 | 1,0 | 1,5 | 2,0 | 2,5 | 3,0 | 4,0 | 5,0 |
| Träger | - | - 6,6 | - 4,8 | -13,0 | - 7,4 | - 4,3 | - 5,6 | -11,5 | - 8,9 |
| (+)-S-Ibuprofen | 50 | + 9,5 | +33,5* | +51,2* | +53,9 | +45,5 | +42,3 | +33,7 | +38,2 |
| (-)-R-Ibuprofen | 50 | - 5,7 | + 4,0 | + 4,5 | + 0,5 | - 4,7 | - 7,0 | - 2,3 | - 3,4 |
| (+)-Ibuprofen | 50 | + 6,4 | + 8,4 | +15,7 | +15,7 | + 8,5 | +18,1 | + 9,7 | +17,0 |
| Acetylsalicylsäure | 100 | +35,8 | +56,6* | +41,4 | +80,4 | +74,6 | +72,8 | +66,1 | +59,8 |

Statistische Analyse, unter Verwendung des Mann-Whitney U-Tests durchgeführt
* P<0,05 im Vergleich zum Träger

Fig. 1

Prozentuale Veränderung der Schwellspannung, die erforderlich ist, um eine Reizreaktion bei weiblichen Rhesusaffen zu erzielen, die oral mit (S)-(+)-Ibuprofen, (R)-(-)-Ibuprofen, (±)-Ibuprofen, Acetylsalicylsäure bzw. mit dem Träger behandelt wurden:

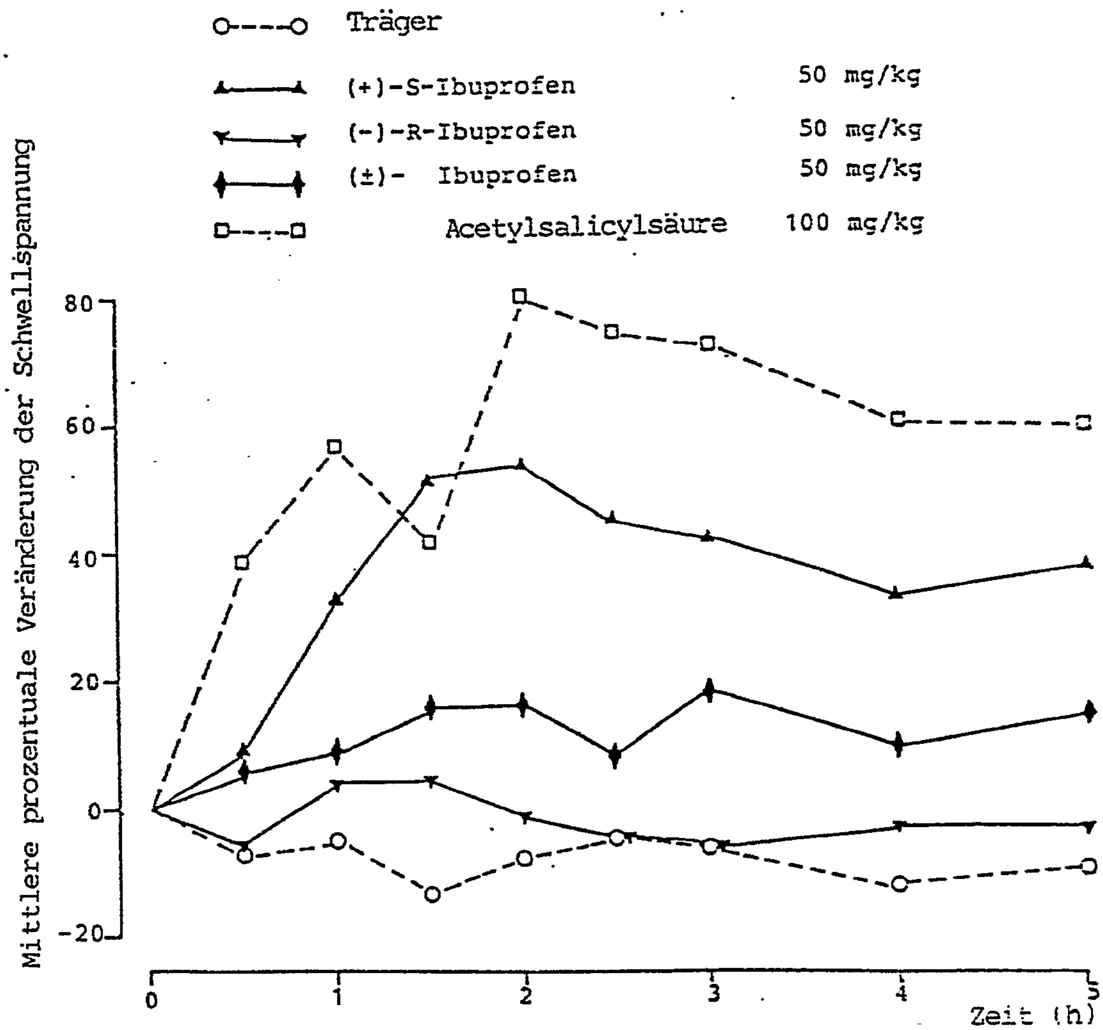


In einem Pilotversuch wurde die Humanpharmakokinetik der Ibuprofenformen untersucht. Die maximalen Plasmaspiegel der wirksamen (S)-(+)-Form liegen nach peroraler Gabe des (S)-(+)-Enantiomeren durchschnittlich 4 bis 5-fach höher als nach Gabe des (R)-(-)-Enantiomeren.Innerhalb der ersten 4 bis 6 Stunden nach Applikatin sind die Flächen unter den Plasmaspiegelkurven (AUC) der (S)-(+)-Form nach Gabe des (S)-(+)-Enantiomer ca. um den Faktor 2,5 größe und als nach Gabe des (R)-(-)-Enantiomeren. Erst danach werden nach Gabe der (R)-(-)-Form gleich hohe bis höhere Spiegel erreicht als nach Gabe der (S)-(+)-Form. Da diese Spiegel bei weniger als 10% der Maximalspiegel liegen, wird kein Beitrag mehr zur erwünschten Wirkung erzielt.

Somit kann auch aus pharmakokinetischer Sicht erklärt werden, daß innerhalb der bekannten Wirkdauer von Ibuprofen (ca. 6 Stunden) von der (R)-(-)-Form keine Wirksamkeit erwartet werden kann.

**Patentansprüche**

1. Ibuprofen enthaltendes Arzneimittel, bei dem Ibuprofen nur in der (S)-(+)-Form in den üblichen Träger- und Verbindungsmitteln enthalten ist, dadurch gekennzeichnet, daß es in Retard-Form vorliegt.

2. Arzneimittel gemäß Anspruch 1, dadurch gekennzeichnet, daß es in einer für eine orale oder rektale oder in Form einer Salbe oder eines Gels für die kutane Applikation geeigneten Zubereitung vorliegt.

3. Arzneimittel gemäß Anspruch 2, dadurch gekennzeichnet, daß es (S)-(+)-Ibuprofen in Retard-Form in Kombination mit nicht retardiertem (S)-(+)-Ibuprofen enthält.

4. Arzneimittel gemäß Anspruch 3, dadurch gekennzeichnet, daß es in Form einer 2-Stufen-Tablette oder als Kapsel vorliegt.

5. Ibuprofen enthaltendes Arzneimittel, dadurch gekennzeichnet, daß es (S)-(+)-Ibuprofen in Kombination mit einer Aminosäure als Lösung enthält.

6. Verwendung von (S)-(+)-Ibuprofen in Retard-Form in Kombination mit nicht retardiertem (S)-(+)-Ibuprofen zur Herstellung eines Arzneimittels.

7. Verwendung gemäß Anspruch 6, dadurch gekennzeichnet, daß das (S)-(+)-Ibuprofen in einer Menge eingebracht wird, die weniger als die Hälfte der Menge beträgt, die erforderlich wäre, um mit Ibuprofen in der Racematform die gleiche Wirkung zu erzielen.

**Claims**

1. An ibuprofen-containing medicament containing ibuprofen only in the (S)-(+)-form, in the usual carriers and diluents, characterized in that it is in sustained-release form.

2. Medicament according to claim 1 characterized in that it is in the form of a preparation suitable for oral or rectal administration, or in the form of a salve or gel for cutaneous administration.

3. Medicament according to claim 2, characterized in that it contains (S)-(+)-ibuprofen in sustained-release form in combination with (S)-(+)-ibuprofen in non-retarded form.

4. Medicament according to claim 3 characterized in that it is present in the form of a two-stage tablet or as a capsule.

5. An ibuprofen-containing medicament, characterized in that it contains (S)-(+)-ibuprofen in combination with an amino acid as a solution.

6. Use of (S)-(+)-ibuprofen in sustained-release form in combination with (S)-(+)-ibuprofen in non-sustained release form for the preparation of a medicament.

7. Use according to claim 6, characterized in that (S)-(+)-ibuprofen is used in an amount which is less than half the amount of racemic ibuprofen which would be needed to obtain the same effect.

**Revendications**

1. Médicament contenant de l'ibuprofène, dans lequel l'ibuprofène ne figure que sous la forme (S)-(+) dans les substances support et les liants usuels, caractérisé en ce qu'il se présente sous la forme retard.

2. Médicament selon la revendication 1, caractérisé en ce qu'il se présente sous la forme d'une préparation appropriée pour l'administration orale ou rectale, ou sous la forme d'un onguent ou d'un gel pour l'administration cutanée.

3. Médicament selon la revendication 2, caractérisé en ce qu'il contient de l'ibuprofène (S)-(+) sous forme retard en combinaison avec de l'ibuprofène (S)-(+) non retardé.

4. Médicament selon la revendication 3, caractérisé en ce qu'il se presente sous la forme d'un comprimé à deux stades ou d'une capsule.

5. Médicament contenant de l'ibuprofène, caractérisé en ce qu'il contient de l'ibuprofène (S)-(+) en combinaison avec un acide aminé, sous forme de solution.

6. Utilisation de l'ibuprofène (S)-(+) sous forme en combinaison avec l'ibuprofène (S)-(+) non retardé, pour la fabrication d'un médicament.

7. Utilisation selon la revendication 6, caractérisée en ce que l'ibuprofène (S)-(+) est introduit en une quantité qui est inférieure à la moitié de la quantité qui serait nécessaire pour produire une activité équivalente avec l'ibuprofène sous forme de racémate.